(19) Europäisches Patentamt
European Patent Office
Office européen des brevets

(11) Veröffentlichungsnummer: **0 219 737**
**B1**

(12) # EUROPÄISCHE PATENTSCHRIFT

(45) Veröffentlichungstag der Patentschrift: **23.05.90**

(51) Int. Cl.⁵: **A61K 31/62**

(21) Anmeldenummer: **86113593.7**

(22) Anmeldetag: **02.10.86**

(54) **Kombinationspräparat.**

(30) Priorität: **09.10.85 DE 3535947**

(43) Veröffentlichungstag der Anmeldung:
**29.04.87 Patentblatt 87/18**

(45) Bekanntmachung des Hinweises auf die Patenterteilung:
**23.05.90 Patentblatt 90/21**

(84) Benannte Vertragsstaaten:
**AT BE CH DE ES FR GB IT LI NL SE**

(56) Entgegenhaltungen:
**EP-A- 0 117 403**
**DE-A- 2 845 220**
**DE-A- 3 124 699**

(73) Patentinhaber: **BASF Aktiengesellschaft, Carl-Bosch-Strasse 38, D-6700 Ludwigshafen(DE)**

(72) Erfinder: **Koenig, Horst, Prof. Dr., Pariser Strasse 4, D-6700 Ludwigshafen(DE)**

**EP 0 219 737 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die Erfindung betrifft ein neues Kombinationspräparat zur Behandlung von Durchblutungsstörungen auf thromboembolischer Grundlage.

Es ist bekannt (vgl. DE-OS 2.845.22O, 3.124.699, EP-OS 117.4O3), daß bestimmte Pyridazinon-Derivate eine thrombocytenaggregationshemmende Wirung besitzen. Es ist weiter bekannt, daß Acetylsalicylsäure aufgrund seiner thrombocytenaggregationshemmenden Wirkung zur Prophylaxe von thromboembolischen Erkrankungen verwendet wird (vgl. die Liste Pharmindex III/84, Colfarit®).

Es wurde nun gefunden, daß sich die Wirkung von Acetylsalicylsäure durch Pyridazinonderivate stark erhöhen läßt.

Gegenstand der Erfindung ist ein Arneimittel, dadurch gekennzeichnet, daß es Acetylsalicylsäure und ein Pyridazinon-Derivat der Formel I

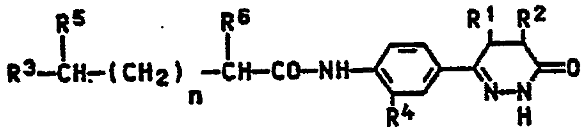

I,

worin

$R^1$ in Wasserstoffatom oder eine Methylgruppe ist,
$R^2$ ein Wasserstoffatom oder zusammen mit $R^1$ eine Methylen- oder Ethylengruppe darstellt,
$R^3$
a) eine Gruppe der Formel

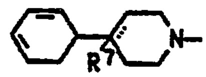

worin die gestrichelte Linie eine zusätzliche Bindung bedeuten kann und $R^7$ gegebenenfalls ein Wasserstoffatom oder einen $C_{1-4}$-Acylrest darstellt, oder
b) eine Gruppe der Formel

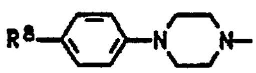

,

worin $R^8$ ein Wasserstoffatom oder eine $C_1$-$C_4$-Acrylgruppe darstellt, oder
c) den 1,3-Tetrahydroisochinolin-2-yl-Rest bedeutet,
$R^4$ ein Wasserstoffatom oder zusammen mit $R^3$ eine direkte Bindung oder eine Methylengruppe darstellt,
$R^5$ ein Wasserstoffatom oder eine Methylgruppe ist,
$R^6$ ein Wasserstoffatom oder ein Halogenatom bedeutet und
n die Zahl O oder 1 ist,

einer Menge von 1OO-6OO Gewichtsteilen Acetylsalicylsäure und 5-5O Gewichtsteilen der Verbindung I enthält.

In das Arzneimittel kann die Acetylsalicylsäure auch in Form ihrer physiologisch verträglichen Salze eingearbeitet werden. Als solche kommen insbesondere die Alkali- und Erdalkalisalze in Betracht.

Das angegebene Mischungsverhältnis bezieht sich auf die freie Acetylsalicylsäure. Der bevorzugte Bereich des Mischungsverhältnisses liegt bei etwa 3OO Teilen Acetylsalicylsäure und 1O Teilen der Verbindung I.

Die überlegene Wirkung der neuen Kombination läßt sich durch Bestimmung der Thrombocytenaggregation in folgenden Versuchen zeigen:

1. Thrombozytenreiches Plasma wird durch Zentrifugation (3OO g, 1O min Dauer bei 4°C) von venösem Citratblut gewonnen. Die photometrische Messung der Thrombozytenaggregation erfolgt unter Zusatz von $MgCl_2$ (Endkonzentration 1O mmol/l) und von Collagen Stago (Endkonzentration O,O2 mg/ml) im Born-Aggregometer Mk 3. Als Aggregationsmaß findet die maximale Extinktionsänderung/sec Verwendung.
Die Prüfung der aggregationshemmenden Wirksamkeit der Substanzen wird nach einer Inkubationszeit von 1O min vorgenommen.
Als EC 5O % wird die Konzentration bestimmt, welche eine 5O %ige Hemmung der Aggregation verursacht.

2. Beagle-Hunde (1O bis 15 kg Körpergewicht) erhalten die Substanzen bzw. die Kombination oral. Vor sowie 2 und 4 h nach der Substanzabgabe wird venöses Blut entnommen, dieses durch Citrat-Zusatz ungerinnbar gemacht und daraus durch anschließende Zentrifugation (300 g, 10 min. Dauer bei 4°C) thrombozytenreiches Plasma gewonnen. Zugabe von Adrenalin (Endkonzentration $5 \times 10^{-8}$ mol/l) und Kollagen (Endkonzentration $2 \times 10^{-3}$ g/l)zu thrombozytenreichem Plasma löst eine Aggregation aus, die als Extinktionslösung im Born-Aggregometer MK 3 gemessen wurde. Als Aggregationsmaß findet die maximale Extinktionsänderung pro Sekunde Verwendung. Aus dem Vergleich der Werte vor und nach Gabe der Substanz wurde die prozentuale Hemmung der Aggregation ermittelt.

In Versuch 2 wurden folgende Ergebnisse erreicht:

| Substanz | Dosis (mg/kg) | Aggregationshemmung (%) |
|---|---|---|
| Amipizone (EP-OS 117.403, Beispiel 13) | 0,1 | 10 |
| Acetylsalicylsäure | 1,0 | 0 |
| Amipizone + Acetylsalicylsäure | 0,1 + 1,0 | 16 |

Die neue Kombination eignet sich zur Behandlung und Prophylaxe von Erkrankungen, die durch Thrombocytenaggregation ausgelöst werden. Dazu gehören thromboembolische Erkrankungen im Bereich von Herz, Gehirn und peripherem, arteriellem Gefäßsystem. Solche Erkrankungen sind beispielsweise Herzinfarkt, Schlaganfall sowie obliterierende Arteriosklerose.

Die neue Kombination ist weiterhin geeignet zur Behandlung und Prophylaxe von Erkrankungen, die durch die Freisetzung vasoaktiver Stoffe aus aggregierenden Thrombocyten hervorgerufen werden, wie Migräne, vasospastische Angina und Morbus Raynaud. Sie ist auch nützlich zur Verhütung von Komplikationen bei chirurgischen Maßnahmen, wie Gefäßprothesen und Shunts.

Die erfindungsgemäße Kombination kann in üblicher Weise oral verabfolgt werden.

Die Dosierung hängt vom Alter, Zustand und Gewicht des Patienten sowie von der Applikationsart ab. In der Regel beträgt die tägliche Dosis zwischen etwa 5 und 50 mg, vorzugsweise etwa 10 mg, Verbindung I und zwischen etwa 30 und 600, vorzugsweise etwa 300 mg, Acetylsalicylsäure pro Patient und Tag.

Die neue Kombination kann in den gebräuchlichen galenischen Applikationsformen fest oder flüssig angewendet werden, z.B. als Tabletten, Filmtabletten, Kapseln, Pulver, Granulate, Dragees, Pellets, Retardpellets oder Lösungen. Diese werden in üblicher Weise hergestellt. Die Wirkstoffe können dabei mit den üblichen galenischen Hilfsmitteln wie Tablettenbindern, Füllstoffen, Konservierungsmitteln, Tablettensprengmitteln, Fließreguliermitteln, Weichmachern, Netzmitteln, Dispergiermitteln, Emulgatoren, Lösungsmitteln, Retardierungsmitteln und/oder Antioxidantien verarbeitet werden (vgl. H. Sucker et al: Pharmazeutische Technologie, Thieme-Verlag, Stuttgart, 1978). Die so erhaltenen Applikationsformen enthalten den Wirkstoff normalerweise in einer Menge von 10 bis 99 Gewichtsprozent.

Beispiel 1

Auf einer Tablettenpresse werden in üblicher Weise Tabletten folgender Zusammensetzung gepreßt:
300 mg Acetylsalicylsäure
5 mg 2-(1,2,3,4-Tetrahydrochinolin-2-on-6-yl)-3,4-diaza-bicyclo-[4.1.0]-heptan-(2)-on(5)
72 mg Maisstärke
13 mg Gelatine
35 mg Milchzucker
25 mg Carboxymethylcellulose
17,5 mg Talcum
3,5 mg Magnesiumstearat

Beispiel 2

In üblicher Weise werden Drageekerne folgender Zusammensetzung hergestellt:
200 mg Acetylsalicylsäure
5 mg 2-(2,3,4,5-Tetrahydro-benzo[b]azepin-2-(1H)-on-7-yl)-3,4-diaza-bicyclo-[4.2.0]-octen-(2)-on-(5)
100 mg Kernmasse

Die Kernmasse besteht aus 9 Teilen Maisstärke, 3 Teilen Milchzucker und 1 Teil ®Luviskol VA 64 (Vinylpyrrolidon-Vinylacetat-Mischpolymerisat 60 : 40, vgl. Pharm. Ind. 1962, 586). Die Verzuckerungsmasse besteht aus 5 Teilen Rohrzucker, 2 Teilen Maisstärke, 2 Teilen Calciumcarbonat und 1 Teil Talk.

Die erhaltenen Kerne werden anschließend mit einer Verzuckerungsmasse folgender Zusammensetzung überzogen:
5 Teile Saccharose
2 Teile Maisstärke
2 Teile Calciumcarbonat
1 Teil Kalk

Beispiel 3

Zur Herstellung von Filmtabletten werden die gemäß Beispiel 2 hergestellten Drageekerne mit einem Filmüberzug folgender Zusammensetzung überzogen:

| | | | |
|---|---|---|---|
| Tylose | 0,7 % | Maisstärke | 2,0 % |
| (R)Kollidon 25 (PVP) | 0,4 % | Calciumcarbonat | 3,5 % |
| Saccharose | 70,0 % | Gummi arabicum | 2,5 % |
| hochdisperse Kieselsäure | 1,4 % | Titandioxid + | |
| Talcum | 11,0 % | Farbstoffe | 8,5 % |

Beispiel 4

Es werden in Steckkapseln abfüllbare Retardpellets hergestellt, wobei jede Komponente für sich pelletiert und die Retardpellets anschließend gemischt oder nacheinander in die Kapseln gefüllt werden.
Zusammensetzung der Pellets pro Dosis.
Retardpellets, Verbindung I:
400 mg Acetylsalicylsäure
60 mg Cellulosepulver
5 mg Maisstärke
10 mg Talkum
35 mg Ethylcellulose
Retardpellets, Verbindung II:
5 mg 6-[p-3-(4-Phenyl-1-piperidyl)-propionylamino-phenyl]-4,5-dihydro-5-methyl-3(2H)-pyridazinon
20 mg Cellulosepulver
10 mg Maisstärke
10 mg Ethylcellolose

Beispiel 5

Es werden in Steckkapseln abfüllbare Pellets hergestellt mit folgender Zusammensetzung:
300 mg Acetylsalicylsäure
10 mg 6-[p-3-(4-Phenyl-1-piperazinyl)-propionylamino-phenyl]-4,5-dihydro-5-methyl-3(2H)-pyridazinon
80 mg Cellulosepulver
30 mg Maisstärke
5 mg ®Kollidon 30 (PVP)
20 mg ®Eudragit S (Polymerisat aus Methylacrylsäure und Methacrylsäureester)
15 mg Talkum

## Patentansprüche

1. Arzneimittel, dadurch gekennzeichnet, daß es Acetylsalicylsäure und ein Pyridazinon-Derivat der Formel I

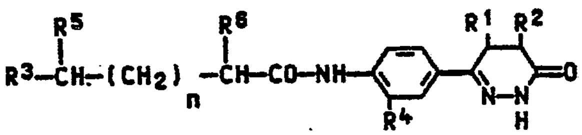 I,

worin
$R^1$ ein Wasserstoffatom oder eine Methylgruppe ist,
$R^2$ ein Wasserstoffatom oder zusammen mit $R^1$ eine Methylen- oder Ethylengruppe darstellt,
$R^3$
a) eine Gruppe der Formel

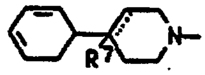

worin die gestrichelte Linie eine zusätzliche Bindung bedeuten kann und $R^7$ gegebenenfalls ein Wasserstoffatom oder einen $C_{1-4}$-Acylrest darstellt, oder
b) eine Gruppe der Formel

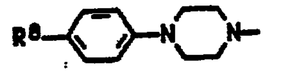

worin $R^8$ ein Wasserstoffatom oder eine $C_{1-4}$-Acylgruppe darstellt, oder
c) den 1,3-Tetrahydroisochinolin-2-yl-Rest bedeutet,
$R^4$ ein Wasserstoffatom oder zusammen mit $R^3$ eine direkte Bindung oder eine Methylengruppe darstellt,
$R^5$ ein Wasserstoffatom oder eine Methylgruppe ist,
$R^6$ ein Wasserstoffatom oder ein Halogenatom bedeutet und
n die Zahl O oder 1 ist,
in einer Menge von 30-600 Gewichtsteilen Acetylsalicylsäure und 5-50 Gewichtsteilen der Verbindung I enthält.

2. Verfahren zur Herstellung eines Arzneimittels gemäß Anspruch 1, dadurch gekennzeichnet, daß man 5-50 Gewichtsteile einer Verbindung der Formel I gemäß Anspruch 1 mit 30-600 Gewichtsteilen Acetylsalicylsäure mischt und die so erhaltene Mischung zu einer galenischen Applikationsform verarbeitet.

## Claims

1. A drug which contains acetylsalicylic acid and a pyridazinone derivative of the formula I

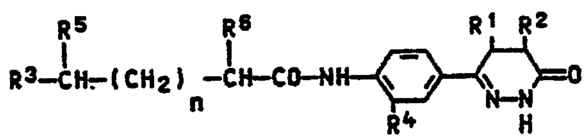

I,

where $R^1$ is hydrogen or methyl, $R^2$ is hydrogen or, together with $R^1$, forms methylene or ethylene, $R^3$ is
a) a group of the formula

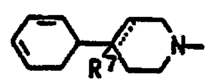

where the dashed line may be an additional bond and, where relevant, $R^7$ is hydrogen or $C_1$–$C_4$-acyl, or
b) a group of the formula

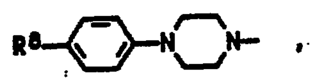

where $R^8$ is hydrogen or $C_1$–$C_4$-acyl, or
c) 1,3-tetrahydroisoquinolin-2-yl,
$R^4$ is hydrogen or, together with $R^3$, forms a direct bond or methylene, $R^5$ is hydrogen or methyl, $R^6$ is hydrogen or halogen and n is 0 or 1, acetylsalicylic acid being present in an amount of 30–600 parts by weight and the compound I in an amount of 5–50 parts by weight.

2. A process for the preparation of a drug as claimed in claim 1, wherein 5–50 parts by weight of a compound of the formula I as claimed in claim 1 are mixed with 30–600 parts by weight of acetylsalicylic acid, and the mixture thus obtained is converted to a pharmaceutical administration form.

## Revendications

1. Médicament, caractérisé par le fait qu'il contient de l'acide acétylsalicylique, en proportions de 30–600 parties en poids, et un dérivé de pyridazinone de formule I, en proportions de 5–50 parties en poids

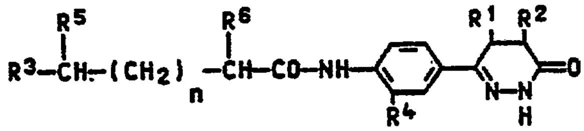

I,

où

$R^1$ est un atome d'hydrogène ou un groupe méthyle,

$R^2$ représente un atome d'hydrogène ou, ensemble avec $R^1$, un groupe méthylène ou éthylène,

$R^3$

a) représente un groupe de formule

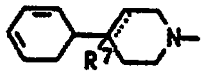

où la ligne pointillée peut signifier une liaison supplémentaire et $R^7$ représente un reste acyle en $C_{1-4}$, ou

b) un groupe de formule

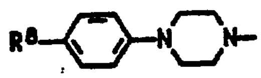

,

où $R^8$ représente un atome d'hydrogène ou un groupe acyle en $C_{1-4}$, ou

c) signifie le reste 1,3-tétrahydroisoquinolin-2-yle,

$R^4$ représente un atome d'hydrogène ou, ensemble avec $R^3$, une liaison directe ou un groupe méthylène,

$R^5$ est un atome d'hydrogène ou un groupe méthyle,

$R^6$ est un atome d'hydrogène ou un atome halogène et

n est le nombre 0 ou 1.

2. Procédé de préparation d'un médicament selon la revendication 1, caractérisé par le fait que l'on mélange 5–50 parties en poids d'un composé de formule I selon la revendication 1 avec 30–600 parties en poids d'acide acétylsalicylique et l'on met le mélange ainsi obtenu sous forme d'application galénique.